# EUROPEAN PATENT APPLICATION

(11) **EP 1 431 879 A2**
(43) Date of publication of application: **23.06.2004**
(21) Application number: 03104634.5
(22) Date of filing: 11.12.2003
(51) Int. Cl.: G06F 17/00, A61B 5/0245, A63B 22/00

(54) **Setting of heart rate limit in heart rate monitor**

(30) Priority: 18.12.2002 FI 20022225
(71) Applicant: Polar Electro Oy, 90440 Kempele (FI)
(72) Inventor: Nissilä, Seppo, 90550, Oulu (FI)
(74) Representative: Niemi, Hakan

(57) **Abstract**

The invention relates to a method and arrangement for measuring heart rate, comprising: means for inputting (352) a heart rate limit for an exercise; means for measuring (302-304) the user's heart rate during the exercise. The arrangement further comprises means for changing (350) the heart rate limit during the exercise on the basis of a predetermined change criterion associated with the exercise.

## Description

### FIELD

The field of application of the invention comprises heart rate monitors used for measuring heart rate in connection with exercising and sports. The invention particularly relates to the setting of heart rate limits in a heart rate monitor.

### BACKGROUND

The evaluation and planning of the intensity of an exercise is important both to a fitness enthusiast as well as a competing athlete. A reliable method for evaluating the intensity is to monitor the frequency of heartbeat, i.e. heart rate, measured from the person's body. Depending on the target intensity set for the exercise, the user may aim at maintaining the heart rate within a desired range. With heart rate monitors exercising can be arranged to take place within a desired heart rate zone by setting heart rate limits. A fixed lower limit and/or upper limit may be set for exercising or for performing in a competition, heartbeat values outside the heart rate zone determined by the set limits causing an alarm that is given in the form of a sound signal, for example.

Prior art heart rate monitors, however, involve a problem in that the heart rate limit settings do not take into account an on-going exercise as a whole and do not adapt to the exercise or to changes the exercise causes in the user's stress level.

### SUMMARY

It is an object of the invention to provide a solution for flexible setting of heart rate limits in connection with an exercise. The invention relates to a method for setting heart rate limits in connection with an exercise, the method comprising inputting a heart rate limit for the exercise, measuring the user's heart rate during the exercise. In the method the heart rate limit is changed during the exercise on the basis of a predetermined change criterion associated with the exercise.

The invention further relates to an arrangement for measuring heart rate, comprising: means for inputting a heart rate limit for an exercise, means for measuring the user's heart rate during the exercise. The arrangement comprises means for changing the heart rate limit during the exercise on the basis of a predetermined change criterion associated with the exercise.

Preferred embodiments of the invention are disclosed in the dependent claims.

The invention aims at providing a solution that enables heart rate limits set in a heart rate monitor to adapt to an exercise carried out by a user. In a sports performance of a long duration the user's average heart rate level often rises as the exercise continues and the user's stress level rises. In current heart rate monitors, problems arise easily in connection with the planning of a marathon, for example, because towards the end of the race and as the stress level rises, heart rate may easily increase to a level that exceeds heart rate limits set in advance for the race.

In the solution of the invention, the heart rate limit is changed during the performance. In this context, the term 'heart rate limit' refers to a limit value of heart rate, expressed by a unit of 'heartbeats per minute', for example. A lower heart rate limit is a limit above which the user tries to maintain the heart rate during the exercise, an upper heart rate limit, in turn, being a heart rate value which the heart rate should not exceed during the exercise. The invention makes it possible to change both of these values, the lower and the upper limit, or only one of them. The heart rate monitor may give an alarm as a sound signal, for example, if the heart rate value is no longer within the heart rate zone defined by the lower and the upper limit.

A heart rate limit may be changed on the basis of a change criterion associated with the heart rate limit. The criterion may be exercise duration, user's stress level or heart rate level, for example. According to an embodiment, the user may be asked to provide, prior to the exercise, input data, such as lower heart rate limit, target heart rate, and exercise duration. On the basis of these data, the heart rate monitor is capable of determining exercise-specific heart rate limits. The heart rate limits, i.e. the lower and the upper limit, for the exercise may be determined for example such that they rise consistently during the exercise at intervals of three minutes until the desired heart rate level is achieved at the end of the exercise. A mathematical model to be employed for determining the heart rate limits may be for example a linear model, an exponent model, or a quadratic curve. This means that the heart rate limits may also lower during the exercise or competition, if the exercise profile so requires.

An advantage of the invention is an improved solution for a heart rate monitor, which enables an exercise or a competition performance of a long duration to be planned better than before.

### LIST OF FIGURES

In the following, the invention will be described in greater detail with reference to the accompanying drawings, in which:
Figure 1 illustrates an embodiment of the method of the invention;
Figure 2 illustrates a function for changing a heart rate limit; and
Figure 3 illustrates an implementation of a heart rate transmitter and a wrist-worn device.

### DESCRIPTION OF EMBODIMENTS

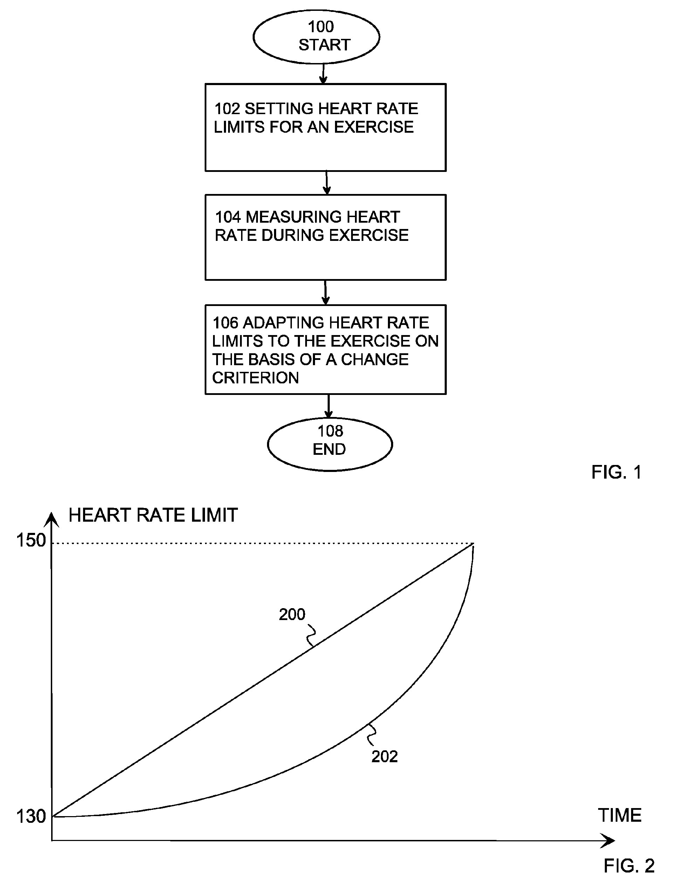

In the following, the invention will be described in connection with some preferred embodiments and with reference to the accompanying figures. Figure 1 illustrates an embodiment of the invention. In initial method step 100, a user has set a heart rate monitor ready for measuring for example by placing a transmitter electrode belt acting as a transmitter around his chest, a receiver unit being ready to receive heart rate information from the transmitter. In method step 102 the user sets initial heart rate limits for the exercise to be carried out. The purpose of the heart rate limits is to control that the exercise is carried out at the desired level. For a person exercising for fitness or for health, one important object of control is to make sure that the intensity of the exercise does not rise to a level that is hazardous for health. For weight management it is important to control that the heart rate level remains within a zone optimal for fat burning. For a competing athlete it is essential to control during the competition that the stress level remains optimal during the entire performance to enable the best possible result to be achieved.

In a performance of a long duration, such as a marathon, the stress level of the athlete tends to rise as a function of time. Although the athlete may feel that he is carrying out the performance at constant intensity, the fact that the user's average heart rate value increases as the performance continues indicates that his stress level rises. During a performance of several hours the average rate may increase by tens of percents. It is thus obvious that fixed heart rate limit settings are not optimal for controlling the intensity of a physical performance. If the user plans to carry out a marathon at a heart rate zone of 120 - 140, for example, then in practice towards the end of the race the heart rate level may be around 160, even if intensity as experienced by the user had not changed significantly. According to prior art solutions, the above situation could have been taken care of by setting the initial heart rate limits between 120 and 165, for example, but these limits would not have served the desired purpose, i.e. they would have functioned poorly during the initial phase of the performance when the stress level is still low. The user is typically responsible for planning and carrying out an exercise or a competition of a long duration such that at first the performance is carried out at a lower heart rate level and as the performance continues, the heart rate level is allowed to rise and towards the end the performance is carried out at maximum heart rate level.

In method step 104 the heart rate is measured during the exercise. Heart rate monitors of different functioning principles may be used for measuring the heart rate. One alternative is a heart rate monitor consisting of a heart rate transmitter to be fitted around the chest and a wrist-worn receiver. Another solution is based on measuring the pressure acting on the carpal artery. It is also possible to use a heart rate monitor based on optical heart rate measurement.

In step 106 the heart rate limits are adapted to the exercise on the basis of a change criterion. One change criterion that can be applied is exercise duration. The heart rate limit may be raised, for example, by one beat every five minutes. Instead of this kind of linear model, an exponential model, for example, in which the rate of change increases as a function of time, can be used as well. During the first hour, for example, the heart rate limit may be raised by one step every ten minutes, whereas during the second hour an increase of one step is carried out every eight minutes. According to yet another model, it is possible to use a quadratic curve, for example a model of two straight lines, in which case during a first period the heart rate limit is changed on the basis of a first straight line and during a second period on the basis of a second straight line. The first and the second periods may be based on a predetermined target time set by the user and they may both account for a half of the target time.

A second change criterion that can be applied is the user's stress level. The stress level is a variable formed in heart rate monitors and it may be based on a host of input parameters. Among the input parameters that may be applied are, for example, the user's level of fitness, his state of activeness, heart rate during exercise, exercise duration, etc. The stress level can be formulated by means of a neural network model, for example.

Another applicable change criterion is energy consumption. In a heart rate monitor energy consumption can be calculated typically by means of a calculation model based on the heart rate. Also cumulative energy consumption can be used as a change criterion.

A yet further change criterion that can be used is a moving heart rate value, which is simpler than the stress level. For example, an average heart rate can be calculated for the last 15 minutes, and the heart rate limits can then be set according to the calculated average value for example such that the lower limit is 10 heartbeats per minute below the average and the upper limit, correspondingly, 10 heartbeats above the average.

The term 'heart rate limit' used above may refer to both the lower and the upper limit. The same rules of change may apply to both, i.e. the upper limit may be moved at the same rate of change as the lower limit. On the other hand, the rates of change of the heart rate limits may also be different, i.e. the upper limit may change at a higher rate than the lower limit, for example. Moreover, the changing of a heart rate limit may affect only one of the limits, i.e. the lower limit may be kept constant during the entire performance and only the upper limit is changed.

According to an embodiment, the width of the range between the upper and the lower limit is changed as a function of time. The changing of the width/length of the range may involve simultaneous raising or lowering of a heart rate limit or limits, or the length of the range may be changed independently of the raising/lowering of the limits. The zone defined by the lower and the upper limit may be reduced in size, for example, as heart rate increases.

Figure 2 illustrates two ways of changing the heart rate limit. The x-axis represents exercise duration and the y-axis the heart rate value associated with a heart rate limit. The heart rate limit to be changed may be the lower or the upper limit. Linear changing of the heart rate limit is depicted with change function 200 and exponential changing of the limit with curve 202.

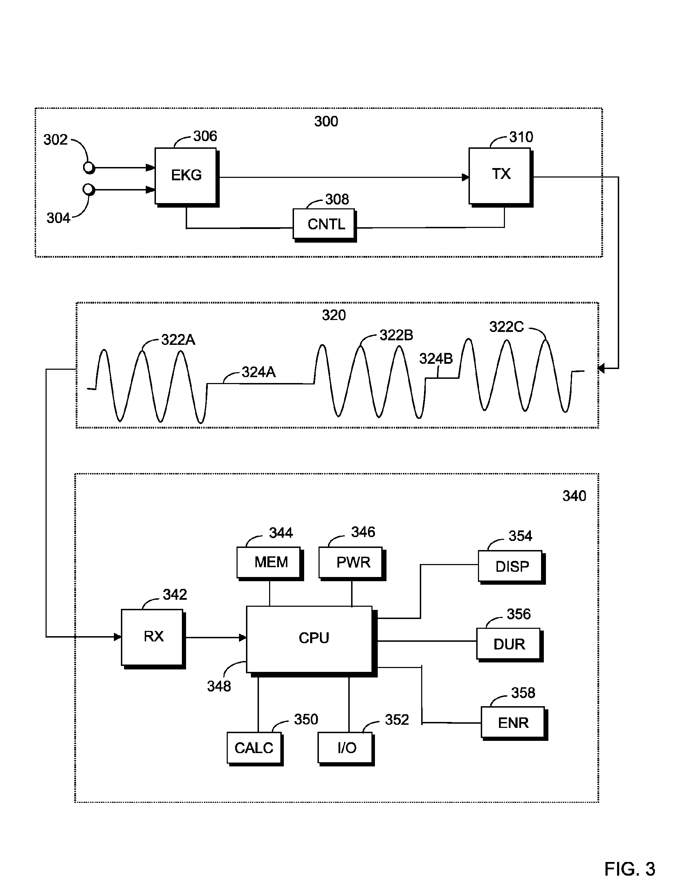

Figure 3 is a block diagram illustrating a heart rate transmitter-receiver pair. The Figure only shows the essential parts of the transmitter, such as an electrode belt 300 to be fitted around the chest, and a wrist-worn receiver 340. A person skilled in the art will find it obvious that they may also comprise other parts than those shown in Figure 3, but it is not significant to describe them in this context.

An electronics unit 308 of the electrode belt 300 receives heart rate information from measurement electrodes 302, 304, which are used for measuring an ECG signal by measuring the difference in potential between the electrodes. The ECG signals are preferably processed, i.e. filtered, amplified and identified, in an ECG detection block 306 using prior art methods to allow heartbeats to be identified from the signal. The heartbeats are identified on the basis of a QRS complex distinguishable from a heart signal, the letters Q, R and S referring to potential phases caused in an electric signal by electric activation of the heart. The QRS identification can be carried out in the ECG detection block 306 by means of a matched filter, for example, in which a model complex is compared with a measured QRS complex and when the comparison exceeds a predetermined threshold value, the measured complex is accepted as a heartbeat. The heartbeat information 320 is transmitted from the electrode belt 300 to the wrist-worn device 340 by means of the transmitter 310 implemented as a coil, for example.

In the heart rate information 320 to be transmitted one heartbeat or one data bit of the heart rate information corresponds for example to one 5 kHz burst 322A or a heartbeat may correspond to a group of several bursts 322A, 322B, 322C. Intervals 324A, 324B between the bursts may be equal or different in length. The heart rate information 320 to be transmitted may consist of heartbeat information, as described above, or calculated heart rate variables, such as an average heart rate or a heart rate distribution, can be formulated from the heartbeats already at the transmitter 300. The calculated variables can naturally be formed also with the wrist-worn device 340 on the basis of the heart rate information. The information 320 may be transmitted inductively or, alternatively, optical or wired transmission may be used. The wrist-worn device 340 comprises receiver means 342, such as a coil. A signal received with the receiver means 342 is supplied to the control electronics 348, which controls and co-ordinates the operation of the electronic parts of the wrist-worn device 340. The control electronics 348 with the associated memory 344 are preferably implemented by means of a general-purpose microprocessor provided with the necessary system and application software, although different hardware implementations are also possible, such as a circuit made up of separate logic components or one or more ASIC circuits (Application Specific Integrated Circuit).

The wrist-worn device 340 comprises a power source 346 for producing electric energy for the electronics unit 348 and a display 354. The received heart rate information 320 and the computer software of the wrist-worn device 340 are stored in the memory 344 of the wrist-worn device 340. The wrist-worn device 340 further comprises a user interface 352 for the heart rate monitor. The user interface is a menu-based hierarchical system, for example, in which push buttons are used for making selections and for activating and deactivating functions, such as heart rate measurement. Information can be supplied to the heart rate monitor via the user interface, and measured or computed information can be obtained as output from the heart rate monitor. The information to be supplied may be for example heart rate information, such as the lower and the upper heart rate limit. The input means 352 may also be used for entering in advance estimated exercise duration prior to the exercise. In addition, parameters relating to the user's physiology, his state of activeness, level of fitness, or the like, may be entered in the heart rate monitor. The input functions of the user interface 352 are implemented using for example voice control, push buttons and/or membrane buttons for making selections and for activating and deactivating functions, such as heart rate measurement.

Information stored in the wrist-worn device 340 may be transferred for further processing via the user interface 352 to an external computer, for example. The output functions of the user interface may be i m-plemented for example by means of a voice transmitter, telecommunications port, infrared transmitter, or the like. Further, the user interface 352 preferably includes means for producing sound signals for example when the time reserved for an exercise has run out or when the heart rate is below or exceeds the set limit. The sound signal for a heart rate that is below the lower limit may be different from the one exceeding the upper limit to allow them to be more easily distinguishable.

The display 354 of the wrist-worn device may be implemented as a liquid crystal display, for example. The display may show for example the function that has been activated by pushing a button in a specific situation of use. In addition, during the exercise, the display shows the user's heart rate. The heart rate limits valid at a given moment may also be displayed.

The heart rate monitor further comprises means for changing the heart rate limit 350. The heart rate changing means 350 may change the heart rate on the basis of information received from the measuring devices 354 measuring the duration of the performance, for example. The heart rate monitor may also comprise means for estimating the stress level 356 of the performance. The stress level estimation means produce a stress level estimate on the basis of the duration of the exercise and the heart rate data obtained during the exercise, for example. Also parameters illustrating the user's fitness and his physiological condition may be used in the evaluation of the stress level. The stress level estimate may be transmitted to the heart rate limit changing means 350, which change the heart rate limits on the basis of a function of change. The wrist-worn device may further comprise means for estimating energy consumption 358. In a heart rate monitor, energy consumption may be estimated using a linear or a non-linear model depending on the heart rate, for example. According to an embodiment, the changing of the heart rate limits depends on energy consumption. In that case the heart rate limits may be changed on the basis of either momentary or cumulative energy consumption.

Although the heart rate monitor disclosed with reference to Figure 3 consists of the electrode belt 300 to be fitted around the chest and the wrist-worn device 340, it is also possible to implement the heart rate monitor as a one-piece wrist-worn device 340. This kind of wrist device comprises pressure sensors for measuring heart rate information from the pressure acting on blood vessels or optical sensors for measuring heart rate optically from the flow of blood in a blood vessel. In a one-piece wrist-worn device heart rate information is transmitted from the sensors to the electronics unit by means of conducting plastics or a wired link, for example.

Although the invention is described above with reference to examples according to the accompanying drawings, it is obvious that the invention is not restricted thereto but can be varied in many ways within the inventive idea disclosed in the accompanying claims.

## Claims

1. A method for setting heart rate limits in an exercise, comprising:
inputting (102) a heart rate limit for the exercise;
measuring (104) the user's heart rate during the exercise,
**characterized by**
changing (106) the heart rate limit during the exercise on the basis of a predetermined change criterion associated with the exercise.

2. A method according to claim 1, **characterized in that** one or more of the following are used as the predetermined change criterion: exercise duration, user's stress level, heart rate during exercise, momentary energy consumption, cumulative energy consumption.

3. A method according to claim 1, **characterized in that** the heart rate limit is changed on the basis of a predetermined change function.

4. A method according to claim 3, **characterized in that** the predetermined change function is a linear model, an exponential model or a quadratic curve.

5. A method according to claim 1, **characterized by**
determining a lower heart rate limit and an upper heart rate limit for the exercise;
carrying out heart rate monitoring by monitoring that the heart rate remains within a heart rate zone that is above the lower heart rate limit and below the upper heart rate limit;
carrying out the changing of the heart rate by changing the extent of the heart rate zone between the lower and the upper limit.

6. A method according to claim 5, **characterized by**
carrying out the changing of the heart rate by reducing the extent of the heart rate zone between the lower and the upper limit as the heart rate level rises.

7. A method according to claim 1, **characterized by**
inputting lower heart rate limit, target heart rate and exercise duration to serve as initial exercise data;
raising the heart rate limit at predetermined intervals during the exercise to allow the target heart rate to be achieved.

8. An arrangement for measuring heart rate, comprising:
means for inputting (352) a heart rate limit for an exercise;
means for measuring (302-304) the user's heart rate during the exercise,
**characterized by** comprising:
means for changing (350) the heart rate limit during the exercise on the basis of a predetermined change criterion associated with the exercise.

9. An arrangement according to claim 8, **characterized in that** the arrangement comprises:
means for measuring the duration of the exercise; and
the duration of the exercise serves as the predetermined change criterion.

10. An arrangement according to claim 8, **characterized in that** the arrangement comprises:
means for estimating the user's stress level; and
the user's stress level serves as the predetermined change criterion.

11. An arrangement according to claim 8, **characterized in that** the heart rate during the exercise or a heart rate variable derived from the heart rate serves as the predetermined change criterion.

12. An arrangement according to claim 8, **characterized in that** the arrangement comprises:
means for estimating the user's energy consumption; and
the energy consumption during the exercise serves as the predetermined change criterion.

13. An arrangement according to claim 8, **characterized in that** the changing means are configured to change the heart rate limit in accordance with a predetermined change function.

14. An arrangement according to claim 13, **characterized in that** the predetermined change function is a linear model, an exponential model or a quadratic curve.

15. An arrangement according to claim 8, **characterized in that**
the inputting means are configured to receive as input data a lower heart rate limit, the heart rate zone above which limit is the zone where the heart rate is to be kept, and an upper heart rate limit, the heart zone below which limit is the zone where the heart rate is to be kept;
the changing means are configured to change the extent of the heart rate zone between the lower and the upper limit.

16. An arrangement according to claim 15, **characterized in that**
the changing means are configured to change the extent of the heart rate zone between the lower and the upper limit on the basis of a change criterion.

17. An arrangement according to claim 8, **characterized in that**
the inputting means are configured to receive the lower heart rate limit, the target heart rate, and the duration of the exercise as input data for the exercise; and
the changing means are configured to raise the heart rate limit during the exercise at predetermined intervals to allow the target heart rate to be achieved.
